# EUROPEAN PATENT SPECIFICATION

(11) **EP 3 884 032 B1**
(45) Date of publication and mention of the grant of the patent: **02.08.2023**
(21) Application number: 19813134.4
(22) Date of filing: 13.11.2019
(51) Int. Cl.: C12M 1/00

(54) **APPARATUS AND METHOD FOR THE GROWTH OF PHOTOSYNTHETIC MICROORGANISMS AND THE BIOFIXATION OF CARBON DIOXIDE THROUGH A HIGH- EFFICIENCY OPTICAL DIFFUSER WITH VARIABLE SPECTRUM AND INTENSITY**
VORRICHTUNG UND VERFAHREN ZUR WACHSTUM PHOTOSYNTHETISCHER MIKROORGANISMEN UND BIOFIXIRUNG VON KOHLENDIOXID DURCH HOCHEFFIZIENTER OPTISCHEN DIFFUSOR MIT EINSTELLBARE SPEKTRUM UND INTENSITÄT
APPAREILS ET MÉTHODE POUR LA CULTIVATION DES MICROORGANISMES PHOTOSYNTHETIQUES ET LA BIOFIXATION DE L'ANHYDRIDE CARBONIQUE PAR DIFFUSEUR OPTIQUE D'HAUTE
ÉFFICIENCE AVEC SPECTRUM ET INTENSITÉ VARIABLES

(30) Priority: 21.11.2018 IT 201800010479
(43) Date of publication of application: 29.09.2021
(73) Proprietor: EVERBLOOM SRL, 10148 Torino (IT); Arcobaleno Cooperativa Sociale, 10148 Torino (IT); MEG S.r.l., 20154 Milano (IT)
(72) Inventor: RIGGIO, Vincenzo Andrea, 10129 Torino (IT); SANTORO, Piero, 20154 Milano (IT); PASSARELLI, Fabio, 10148 Torino (IT)
(74) Representative: Mola, Edoardo
(86) International application number: PCT/IB2019/059732
(87) International publication number: WO 2020/104895

(56) References cited:
- WO-A1-2007/070452
- WO-A1-2008/131019
- WO-A2-2010/014010
- US-A1- 2014 073 035
- US-A1- 2017 101 619

## Description

### TECHNICAL FIELD

The present invention relates to the biotechnological sector and, in particular, concerns an apparatus and method for optimizing the growth processes of photosynthetic organisms such as for example, microalgae and cyanobacteria.

Specifically, the present invention relates to an apparatus and method for the culture of photosynthetic organisms by means of the use of an optical system of artificial lighting.

The most advantageous implementation - that is to say with the best efficiency - of the present invention provides for the combination of the optical system of artificial lighting according to the present invention and the photobioreactor devised by some of the present inventors and described in the European Patent no. EP 2 830 413 B1. The preferred fields of application of the present invention are those typical of biotechnologies, ranging from nutraceuticals to cosmetics, from the production of biofuels to the fixation of greenhouse gases.

### STATE OF THE ART

Traditional technologies for the implementation of processes for the growth of photosynthetic organism involve the use of natural or artificial light.

An example of a technical solution with natural light is described in European Patent no. EP 2 830 413 B1.

The main drawback of the aforementioned solution lies in the fact that the system completely depends on natural light, so that neither intensity nor spectrum can be modified, moreover the photosynthetic activity is confined to the day-time phase only, limiting the overall efficiency of the system.

In the case of artificial light, the light source is normally constituted by fluorescent tubes; examples of this technical solution are described in United States Patent Patent applications no. US 2011/312062 A1 and no. US 2014/073035 A1.

The main drawback of the aforementioned solution lies in the fact that the light incidence is not uniform on the surface of exposure to the photosynthetic organisms under culture from the point of view of the light spectrum (quality of the radiated light) and from the point of view of the irradiated power (quantity of light irradiated), as well as in terms of frequency; in other words, the fluorescent tubes are only able to emit constant light spectrum and frequency, while the power is closely linked to the operating hours and therefore degrades over time.

Consequently, since it is not possible to vary in real-time the composition of the light spectrum, the light power and the power-up frequency, it is not possible to manage the apparatus based on the real needs of the process of growth of photosynthetic organisms.

In particular, photosynthetic organisms, such as microalgae, for example, have specific light absorption requirements and are also dependent on different growth phases.

Thus far, the need to have an apparatus capable of managing both power and light spectrum in a variable and precise way, guaranteeing their uniformity and constancy over time in terms of incident and lighting light spectrum, to production needs varying from time to time is therefore unsatisfied.

The Applicant, with the apparatus and method thereof for the culture of photosynthetic organisms according to the present invention, intends to remedy this lack.

Furthermore, WO-A1-2010140010 i.e. the closest prior art, refers to a system for the cultivation of algae wherein the lighting device is at least partially dipped in the liquid containing algae.

WO-A1-2008131019 shows a container device for a liquid with algae and a lighting device acting inside the container in favor of the algae and energy emitters in the form of panels dipped in the liquid of the container.

### OBJECTS AND SUMMARY OF THE INVENTION

It is an object of the present invention to overcome the drawbacks of the known art linked to the impossibility of realizing processes for the growth of photosynthetic organism based on specific requirements.

In particular, the present invention intends to solve the problem of how to manage both the power and the light spectrum in a variable and precise way, guaranteeing their uniformity in terms of incident and lighting light spectrum, in order to meet the production needs varying from time to time.

More particularly, the present invention intends to solve the problem of how to manage both the power and the light spectrum in a variable and precise way, guaranteeing ideal growth conditions based on the plant species involved.

Such objectives are achieved with the apparatus and method thereof for the culture of photosynthetic organisms according to the present invention which, advantageously and thanks to the presence of an optical device, allows the operating conditions to be varied and adjusted according to the specific requirements.

Advantageously, the present invention allows
- optimizing the growth processes of photosynthetic organisms;
- reducing the growth processes of photosynthetic organisms;
- decreasing power consumption;
- maximizing the bio-absorption efficiency of mineral and gaseous nutrients;
- realizing the contiguity with natural lighting to allow light exposure throughout the day;
- realizing light stress scenarios to stimulate the biosynthesis of high-yielding metabolites.

Specifically, the aforementioned and other objects and advantages of the invention, as will appear from the following description, are achieved with an apparatus for the culture of photosynthetic organisms according to claim 1.

Preferred embodiments and variants of the apparatus according to the present invention form the subject matter of the dependent claims 2 to 7.

Another aspect of the present invention relates to a method for the culture of photosynthetic organisms which uses the apparatus according to the present invention and forms the object of claim 8.

Preferred embodiments and variants of the method according to the present invention form the subject matter of the dependent claims 9 to 13.

It is understood that all the appended claims form an integral part of the present description and that each of the technical features claimed therein is possibly independent and can be used autonomously with respect to the other aspects of the invention.

It will be immediately apparent that countless modifications could be made to what described (for example, regarding shape, sizes, arrangements and parts with equivalent functionalities and usable microorganisms) without departing from the scope of protection of the invention as claimed in the appended claims.

Further advantageous features will become more apparent from the following description of preferred, but not exclusive embodiments provided purely by way of example and not of limitation.

### BRIEF DESCRIPTION OF THE DRAWINGS

The present invention will be described hereinafter by way of some preferred embodiments, provided by way of example and not of limitation, with reference to the accompanying drawings. These drawings illustrate different aspects and examples of the present invention and, where appropriate, similar structures, components, materials and/or elements in different figures are denoted by similar reference numerals.
FIG. 1 is a schematic perspective representation of the general embodiment of the apparatus according to the present invention, with a panel for the culture of photosynthetic organisms and a mono-emissive optical illuminator;
FIG. 2 is a schematic perspective representation of a mono-emissive optical illuminator according to the general embodiment of the present invention illustrated in FIG. 1;
FIG. 3 is a schematic perspective representation of the preferred embodiment of the apparatus according to the present invention, with two panels for the culture of photosynthetic organisms and a bi-emissive optical illuminator;
FIG. 4 is a schematic perspective representation of bi-emissive optical illuminator according to the preferred embodiment of the present invention illustrated in FIG. 3;
FIG. 5 is a schematic front representation of the lighting isolines of a panel for the culture of photosynthetic organisms according to the present invention;
FIG. 6 is a schematic perspective representation of a miniaturized variant of the apparatus according to the present invention;
FIG. 7 is a block diagram which illustrates the method for the culture of photosynthetic organisms by means of an optical illuminator according to the present invention.

### DETAILED DESCRIPTION OF THE INVENTION

While the invention is susceptible to various modifications and alternative constructions, some preferred embodiments are shown in the drawings and will be described in detail hereinbelow.

It should be understood, however, that there is no intention to limit the invention to the specific embodiments illustrated, but, on the contrary, the invention is intended to cover all modifications, alternative constructions, and equivalents that fall within the scope of the invention as defined in the claims.

In the following description, therefore, the use of "for example", "etc.", "or" indicates not exclusive alternatives without any limitation, unless otherwise indicated; the use of "also" means "including, but not limited to" unless otherwise indicated; the use of "includes/comprises" means "includes/comprises but not limited to" unless otherwise indicated.

The apparatus and method of the present invention are based on the innovative concept of using, instead of the usual incandescent and/or fluorescent natural or artificial light sources, an optical device for artificial lighting comprising an optical guide, a plurality of LEDs and a regulation and control panel.

Actually, the Inventors have observed and discovered that the aforesaid optical device for artificial lighting makes it possible to carry out processes of growth and conditioning of photosynthetic organisms based on specific needs.

In the present description, the term "panel" means a planar panel made of transparent material that constitutes a pressure circuit, preferably a coil, with an input point and an output point.

In the present description, the term "photobioreactor" means an apparatus consisting of one or more panels, connection and control hydraulic devices, liquid handling systems, a degassing apparatus and a lighting apparatus as well as a series of control sensors.

In the present description, the term "photosynthetic organisms" means eukaryotic and prokaryotic organisms having microscopic dimensions, with phototrophic (obtaining energy from light) and in general autotrophic metabolism (using inorganic carbon as a carbon source); the photosynthetic microorganisms according to the present definition are generally found, but not exclusively, in an aquatic environment.

The system can be used not only in conditions of autotrophic, but also mixotrophic and heterotrophic growth, for example by carrying out growth in the absence of light and possibly using the optical illuminator as a source for photostimulation aimed at the biosynthesis of metabolites.

In the present description, the term "illuminator" means a system consisting of a series of LEDs, an optical guide used to direct and diffuse the emission, and an electronic control unit for managing the process parameters.

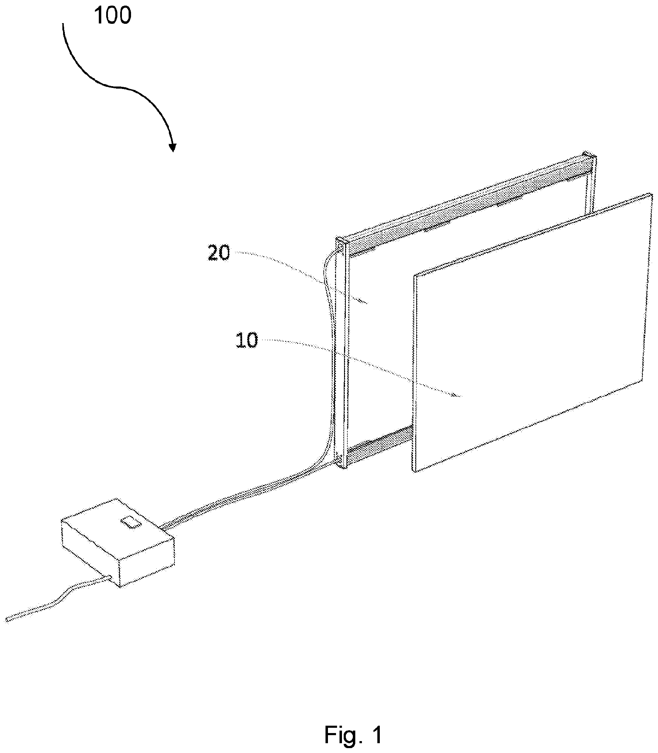

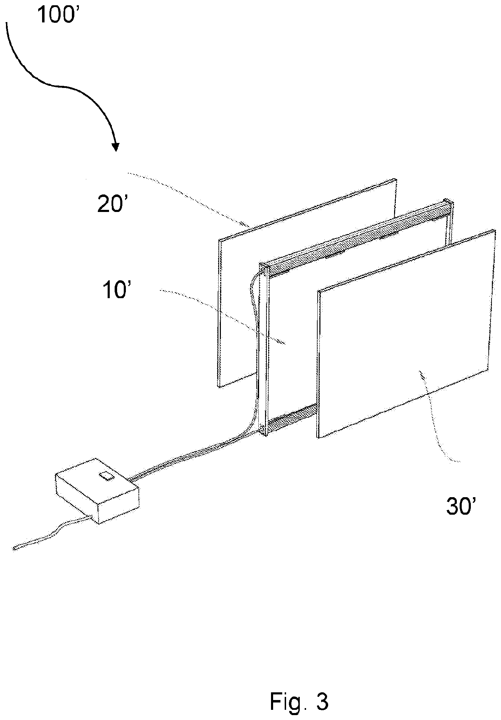

With reference to FIG. 1, which illustrates the general embodiment of the apparatus according to the present invention, and to FIG. 3, which illustrates the preferred embodiment of the apparatus according to the present invention, it can be observed that the apparatus 100, 100' for the culture of photosynthetic organisms 50, 50' comprises
- at least one first panel 20, 20' containing said photosynthetic microorganisms 50, 50' inside it; and
- at least one device for artificial lighting 10, 10' of said photosynthetic microorganisms 50, 50',
wherein said at least one device for artificial lighting 10, 10' is an optical device and comprises
▪ an optical guide 1, 1';
▪ a plurality of LEDs 2, 2'; and
▪ a regulation and control panel 4, 4' of said at least one device for artificial lighting 10, 10',
and wherein said at least one regulation and control panel 4, 4' is suitable to vary both the spectrum, so as to optimize the quality of the light emitted according to the "absorption spectrum" of the growing photosynthetic micro-organism, and the light intensity, so as to obtain emission uniformity, through the optical guide 1, 1'.

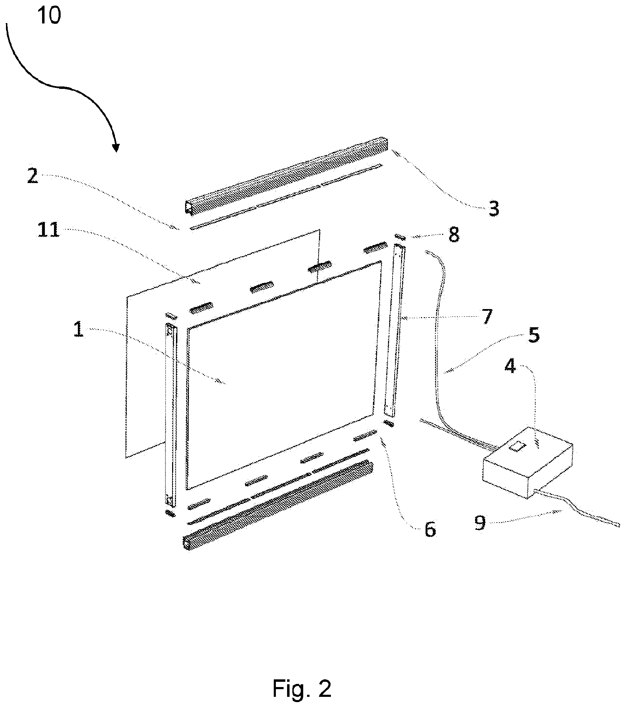

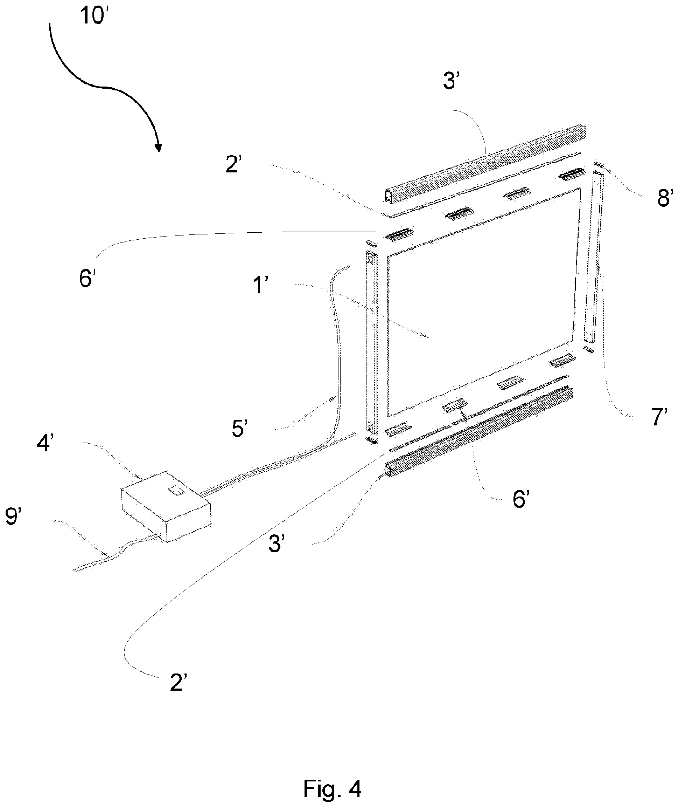

Referring to FIG. 2 and FIG. 4, it is observed that the at least one device for artificial lighting 10, 10' also includes
▪ a pair of profiles 3, 3' to dissipate the thermal energy generated by the LEDs 2, 2';
▪ a plurality of shielded cables 5, 5' for the connection between the LEDs 2, 2' and the regulation and control panel 4, 4';
▪ a pair of supports 6, 6' for centring the optical guide 1, 1';
▪ a pair of uprights 7, 7' to guarantee the stiffness of said at least one device for artificial lighting 10, 10';
▪ closing plugs 8, 8' to close the uprights 7, 7'; and
▪ at least one power supply system 9, 9'.

Still with reference to FIG. 2 and FIG. 4, preferably
▪ the optical guide 1, 1' is an optical guide in polymethylmethacrylate PMMA of optical grade, with light extraction finishing on both large surfaces, the extraction finishing being obtained by means of laser engraving;
▪ the plurality of LEDs 2, 2' consists of 50 power-LEDs divided into four control channels that have the same radiometric flow and are characterized as follows: Ch-1 peak at 660 nm, Ch-2 peak at 455 nm, Ch-3 peak at 730 nm, Ch-4 White 5,000 K;
▪ the pair of profiles 3, 3' are aluminium extrusions; in addition to the function of dissipating the thermal energy generated by the LEDs, the profiles also serve as a housing for the internal wiring and support for the planar photobioreactor;
▪ the regulation and control panel 4, 4' allows to adjust the intensity of every single channel, the power supply stage and the user interface, consequently allowing to create different compositions of the light spectrum and then contextualize them within photoperiods, even dynamic ones; the user interface allows the creation and storage of numerous set-ups by varying, for example, the composition of the spectrum and the dynamic photoperiod;
▪ the shielded cables 5, 5' for the connection between the LEDs 2, 2' and the regulation and control panel 4, 4' are divided into groups of three;
▪ the pair of supports 6, 6', apart from centring the optical guide 1, 1', also serves for fixing the panel;
▪ the uprights 7, 7' are made of aluminium;
▪ the closing plugs 8, 8' are made of plastic; and
▪ the power supply system 9, 9' is a 220-240 V power cable.

Referring to FIG. 2, it can be observed that the at least one device for artificial lighting 10 further comprises a reflector 11 which allows directing the light emission of the first panel 20 towards a single surface.

With particular reference to the general embodiment of the apparatus according to the present invention, illustrated in FIG. 1 and FIG. 2, it is observed that the at least one device for artificial lighting 10 is a mono-emissive optical device.

With particular reference to the preferred embodiment of the apparatus according to the present invention, illustrated in FIG. 3 and FIG. 4, it is observed that the apparatus 100' for the culture of photosynthetic microorganisms further comprises at least a second panel 30' containing said photosynthetic microorganisms inside it and that the at least one device for artificial lighting 10' is a bi-emissive optical device.

Preferably the at least one mono-emissive 10 or bi-emissive optical device 10' for artificial lighting is placed in direct contact with said at least first panel 20, 20'. Preferably the at least one regulation and control panel 4, 4' comprises
▪ a driver for the current driving of the LEDs 2, 2';
▪ a power supply stage;
▪ a programmable microprocessor; and
▪ a digital control interface.

The apparatus 100, 100' according to the present invention allows the spectrum to range between 200 and 900 nm and the light intensity to range between 1% and 100%, the spectrum and the light intensity dynamically varying according to the operating specifications of the device for artificial lighting 10, 10'.

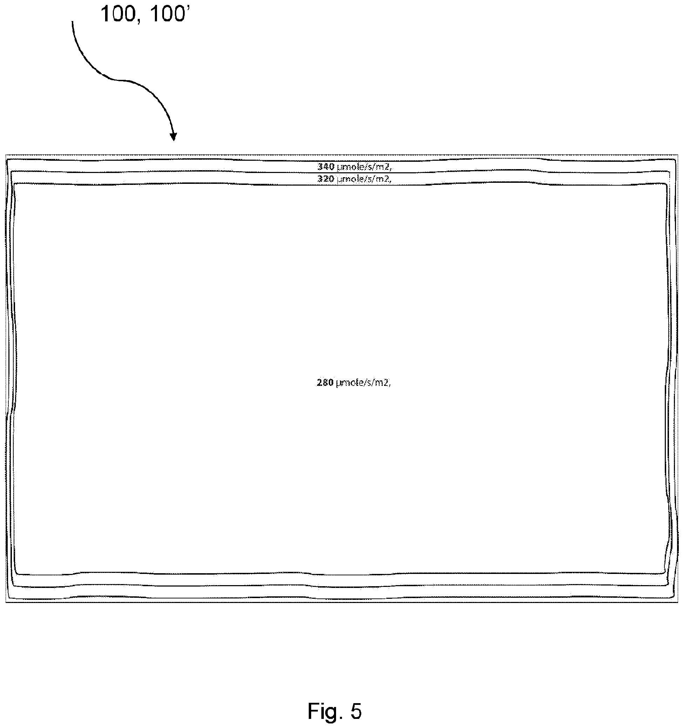

Reference is now made to FIG. 5, which illustrates the lighting isolines of a panel for the culture of photosynthetic microorganisms according to the present invention.

To determine these isolines, a panel having a base of 1,500 mm and a height of 1,000 mm has been considered; the photosynthetic lighting values refer to the incident light on the photobioreactor panel, with a distance between the illuminator and the panel varying from the direct contact up to about 15 mm.

As can be seen from the image, the uniformity of incidence of light is greater than 80% and such lighting has been simulated considering an electric absorption of the illuminator of about 200 W.

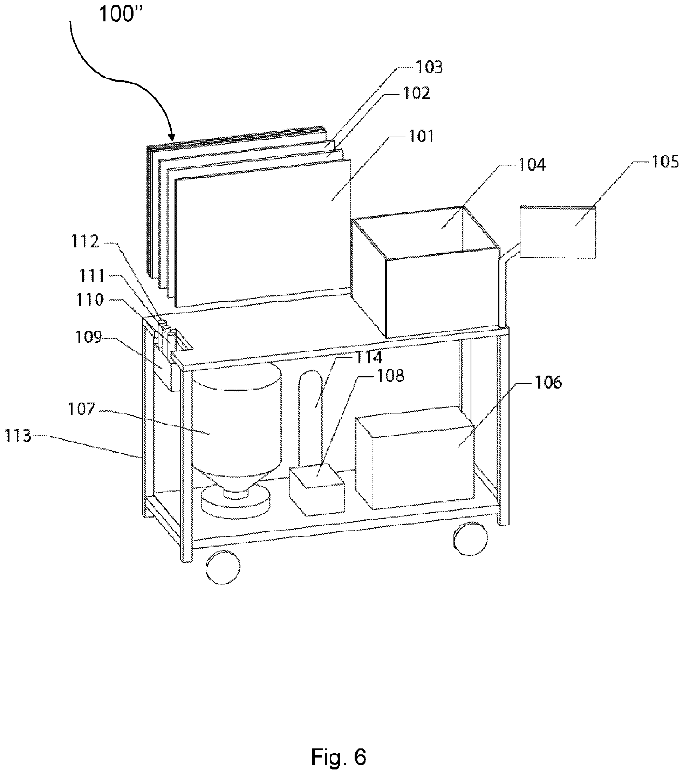

Referring to FIG. 6, a miniaturized variant of the apparatus according to the present invention is illustrated.

In particular, the apparatus 100" comprises:
- a mono-emissive LED panel with four channels, variable spectrum and a quantum sensor for real-time feedback, identified with the reference number 101;
- an alveolar panel, equipped with connection heads and hydraulic system, identified with the reference number 102;
- a bi-emissive LED panel with four channels and variable spectrum, identified with the reference number 103;
- a volumetric illuminator, a LED panel with four channels and variable spectrum equipped with a magnetic platform for mixing the inoculum solution, identified with the reference number 104;
- a hardware interface for control and management, identified with the reference number 105;
- a panel with power stage and PLC peripherals, identified with the reference number 106;
- a tank with a control system for the solution temperature, identified with the reference number 107;
- a delivery pump, identified with the reference number 108;
- a tank for the measurement of the microalgal culture, identified with the reference number 109;
- a first sensor group, identified with the reference number 110;
- a second sensor group, identified with the reference number 111;
- a third sensor group, identified with the reference number 112;
- a support and housing structure of the apparatus, identified with the reference number 113; and
- a gas dispenser with precision flow meter and solenoid valve, identified with the reference number 114.

According to the present invention, the photobioreactor can accommodate all aquatic photosynthetic microorganisms for their growth; by way of example, but not limited to, the aquatic photosynthetic microorganisms comprise green algae (Chlorophyta, for example, Chlorella sp.), diatoms (Chrysophyta), dinoflagellates (Dinophyta), red microalgae (Rhodophyta, for example, Cyanidium sp.), protists (for example, Euglena sp.) and photosynthetic bacteria (cyanobacteria, for example, Spirulina sp.).

The subject invention is characterized by an emitted radiant light power of about 300 µmol/s equally divided between the two emitting surfaces; it is specified that it can intervene in substitution/compensation of natural light in hybrid systems.

The invention is supplied with a voltage of 220-240 V-50 Hz, for maximum absorption of 500 W.

The LED technology used guarantees an expected life-time of about 50,000 hours.

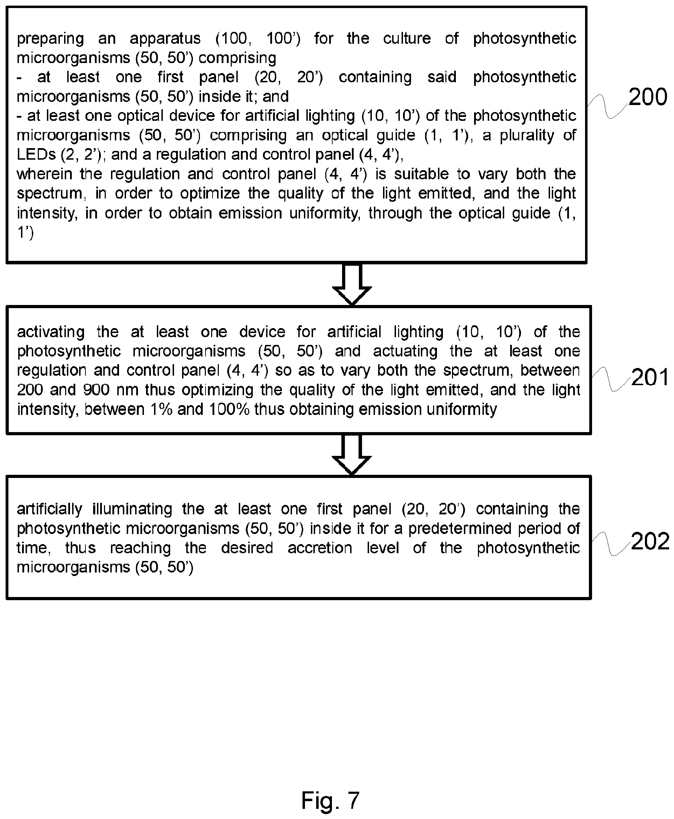

To improve the energy efficiency of the system, it is possible to optimize the optical guide and the thermal management of the variable-spectrum LED illuminators. Furthermore, with reference to FIG. 7, an independent and autonomously usable aspect with respect to the other aspects of the invention is represented by a method for the culture of photosynthetic microorganisms 50, 50' comprising the following steps:
- preparing an apparatus 100, 100' for the culture of photosynthetic microorganisms 50, 50' according to what described above (step 200);
- activating said at least one device for artificial lighting 10, 10' of said photosynthetic microorganisms 50, 50' and actuating said at least one regulation and control panel 4, 4' so as to vary both the spectrum, thus optimizing the quality of the emitted light, and the light intensity, thus obtaining emission uniformity (phase 201); and
- artificially illuminating said at least one first panel 20, 20' containing said photosynthetic microorganisms 50, 50' inside it for a predetermined period of time, thus reaching the desired accretion level of said photosynthetic microorganisms 50, 50' (step 202).

Preferably, the at least one regulation and control panel 4, 4' is operated so as to vary the spectrum between 200 and 900 nm and the light intensity between 1% and 100%, the spectrum and the light intensity dynamically varying according to the operating specifications of the device for artificial lighting 10, 10'.

Preferably, photosynthetic microorganisms are selected from green algae (Chlorophyta), diatoms (Chrysophyta), dinoflagellates (Dinophyta), red microalgae (Rhodophyta), protists, and photosynthetic bacteria such as cyanobacteria.

More preferably, photosynthetic microorganisms are selected from Chlorella sp., Cyanidium sp., Euglena sp. and Spirulina sp.

Preferably, the predetermined time is equal to or greater than two days and the desired level of growth of said photosynthetic microorganisms 50, 50' is equal to or greater than a corresponding growth rate µ equal to 0.01 g/L/day.

According to the present invention the apparatus 100, 100' is also applicable to a panel 10, 10' operating at ambient pressure.

The most advantageous implementation - that is to say with the best efficiency - of the present invention provides, however, for the combination of the optical system of artificial lighting according to the present invention and the photobioreactor devised by some of the same inventors and described in the European Patent no. EP 2 830 413 B1, that is to say with panel 10, 10' which operates under pressure.

More precisely, the present invention allows implementing the growth efficiency of photosynthetic microorganisms within a hydraulic circuit under pressure, artificially illuminated under controlled conditions according to the European Patent no. EP 2 830 413 B1.

Specifically, the present invention allows to provide a compact structure capable of receiving two under-pressure alveolar panels for the growth of photosynthetic microorganisms, interposing a panel of plastic material therebetween, i.e. the optical guide, suitable for the uniform diffusion of both the light spectrum and the light power emitted by different LED illuminators orthogonally positioned with respect to it; the LEDs that constitute the illuminators are able to generate light with different wavelengths of various intensity and varying frequency, allowing to cover the specific process needs of the photosynthetic micro-organism considered.

The combination of the present invention and the photobioreactor described in European Patent no. EP 2 830 413 B1 also comprises:
- an electronic controller, which allows the photoperiod duration to be varied in real-time based on a programmable time sequence;
- a control logic capable of varying the composition of the light spectrum emitted based on the real and specific need for absorption by the growth and conditioning process;
- a control logic capable of varying the frequency of switching on and off of the LED illuminators based on the growth and conditioning process.

The combination of the present invention and the photobioreactor described in European Patent no. EP 2 830 413 B1 improves performances in terms of compactness, versatility (intended as a modification of growth and conditioning parameters), growth rate, nutrient bio-fixation and energy efficiency of the system compared to an incandescent or fluorescent lighting system.

In particular, the aforementioned combination allows to significantly reduce the overall dimensions of the panels which constitute the single module of a planar photobioreactor since between the two panels, which constitute the base unit, the required space is only 1.5 cm instead of 7-10 cm required by the footprint of a normal fluorescent tube illuminator.

Moreover, in this way, the final system results optimally integrated, having a more compact shape, significantly reducing the area occupied per unit of volume. It also increases the ease of plant handling/maintenance and versatility, since the modules can be placed side by side or superimposed on one another, making stacks having a growing capacity of the biomass process according to production needs.

A correct exposure to light allows photosynthetic microorganisms to perform CO₂ bio-fixation processes optimally, making this technology a valuable system for the mitigation of this important greenhouse gas.

The increase in light emission efficiency through the subject invention immediately affects the managing costs of the system, keeping investment costs in the same order of magnitude when compared with more traditional products.

Thanks to the possibility of managing the control logic, which allows to set case-specific light parameters to optimize the growth of photosynthetic microorganisms and to vary them over time, the aforementioned combination guarantees high versatility features and is suitable for autotrophic, mixotrophic and heterotrophic growth, even in conditions that have been prohibitive or never hypothesized to date. An implementation of the aforementioned combination will constitute the raising of the IP sealing level from the current IP 40 to IP 68.

The invention is described below in greater detail with reference to the following Examples, which are to be construed as purely illustrative but without limitation of the present invention.

In particular, examples of embodiments with various types of photosynthetic organisms are described, specifying for each type of organism the configuration and operation of the optical guide.

### Example 1

For the present example, a batch culture of a photosynthetic micro-organism, in particular, a green microalga, for example, *Chlorella* sp., has been considered. Growth has been carried out in totally autotrophic conditions aimed at the bio-fixation of carbon dioxide.

The batch/ autotrophic cultivation of microalgae requires an aqueous growth solution that includes all inorganic minerals and the inlet of a gas flow containing a smaller or larger fraction of carbon dioxide needed to meet the needs for the microalgae replication and growth. Moreover, the initial start of the cultivation of the photobioreactor object of the patent involves the use of a minimum quantity of starting biomass (inoculum), so that the initial concentration of the biomass has an optical density (OD) > 0.08 Abs at 680 nm.

Based on the premises given above, the conduct of such cultivation involves the following steps:
1. Preparation of the microalgal biomass for the photobioreactor inoculum
2. Preparation of the photobioreactor for the conduction of microalgal cultivation in batch/autotrophic mode
3. Conduction of microalgal growth and bio-fixation of carbon dioxide
4. Collection of the biomass produced

### Step 1 - Preparation of the Microalgal Biomass for the Photobioreactor Inoculum

The preparation of the initial inoculum can take place in photobioreactors on a laboratory scale (0.01-20 L). The starting microalga can be taken from an agar growth medium (the maintenance type preferred at long-term or in case of purchase of the species used in international organisms banks). The initial biomass can also be taken from a previously prepared aqueous solution. The laboratory-scale photobioreactor, the aqueous growth solutions, the measuring apparatuses in contact with the growth solution and the gas inlet apparatuses must be properly sterilized before their use. It is preferable to maintain the initial concentration (OD) of the microalga in the photobioreactor, at the time of the inoculation, greater than or equal to 0.100 Abs of Optical Density in order to reduce the initial lag phase in the cell growth. The parameter can be measured, for example, by reading on the spectrophotometer at 680 nm. For the described example, the growth of Chlorella was carried out using the "BBM medium" nutrient solution (Bold 1949, Bischoff & Bold 1963 and subsequent modifications) with controlled lighting with 10-500 µmol m⁻² s⁻¹ Photosynthetic Photon Flow Density (PPFD), with or without day-night cycle.

An appropriate aeration of the solution by introducing a gas mixture containing a certain percentage of carbon dioxide (> 0.04% at 100% v/v) is necessary to supply the microalgae with the gaseous nutrients necessary for the photosynthesis (carbon dioxide) and to remove photosynthetic waste gases (molecular oxygen). To ensure the adequate dissolution of the injected gases and a rapid diffusion among the microalgae in the growth solution, it is necessary to ensure a turbulent motion of the aqueous solution, for example by orbital agitation and/or a porous gas aerator and an air lifting column. The batch cell growth in the laboratory system is monitored at regular intervals by measuring the optical density (OD) as described above. The measured values, if shown on a graph as a function of the culture start time, allow drawing the characteristic cell growth sigmoid chart. At the end of the exponential growth phase, the accumulated biomass will be recovered and transferred to a new photobioreactor with a greater volume by an appropriate dilution (as described above) in a new volume of "BBM medium" growth solution. The cycles of refreshing and increasing the culture volume are repeated until the sufficient quantity of microalgal biomass (OD min > 0.080 abs at 680nm) is obtained to proceed with the inoculation in the photobioreactor covered by the patent.

### Step 2. Preparation of the Photobioreactor for the Conduction of Microalgal Cultivation in Batch/autotrophic Mode

Before proceeding with the inoculation of the microalgae in the photobioreactor object of the patent for the conduction of the cultivation of Chlorella in batch/ autotrophic mode, it is necessary to carry out preliminary preparation operations.

It is necessary to sanitize the closed cultivation system by washing with a disinfectant solution suitable for contact with the photobioreactor construction materials. At the end of the sanitization, it is necessary to remove the residues of the sanitizing solution with repeated washing of the system.

At the time of the inoculation, the photobioreactor is filled with the nutrient solution (BBM medium) up to the desired operating level, with a minimum level of approximately 1/5 of the nominal volume of the photobioreactor. The nutrient solution contains all the inorganic compounds, in optimal quantities, to perform the growth of the microalgae in batch/autotrophic conditions until the stationary growth phase.

### Step 3. Conduction of Microalgal Growth and Bio-fixation of Carbon Dioxide

The microalgal biomass obtained with the previous operations of preparation of the inoculum (Step 1) is introduced into the photobioreactor object of the patent and previously prepared at the start of cultivation, as described in Step 2. During the growth period, it is possible to vary the composition of the nutrient solution to meet specific nutritional needs of the microalga or to modify the growth conditions of the accumulated biomass through the introduction of new nutrients in the photobioreactor.

For the conduction of cultivation in batch/ autotrophic conditions, it is necessary to control and monitor the chemical/physical parameters within the system which, in the example shown, reflect the growth requirements of the selected microalga, Chlorella.

### Lighting:

Growth is carried out under controlled and modulated lighting according to the terms described in the patent. It is possible to adjust the intensity between 10-500 µmol m⁻² s⁻¹ Photosynthetic Photon Flow Density (PPFD), with or without day-night cycle. Furthermore, it is possible to qualitatively modify the lighting spectrum according to the absorption requirements of the cultivated microalga and the desired growth and conditioning needs. In the case described, the growth is carried out using the lighting spectrum characteristic of the white LED with a continuous lighting cycle (24h/24h) in order to keep the microalgae in the photosynthetic phase and obtain the continuous bio-fixation of the carbon dioxide injected into the system.

### Flow of the Nutrient Solution:

The recirculation and handling system of the nutrient solution (hydraulic recirculation pump) is equipped with a speed regulation system. The speed of the nutrient solution recirculation system must be adjusted appropriately in a range that can vary from 5 to 20 L/min in order to guarantee the adequate frequency of exposure of the microalgae to light, the adequate mixing of nutrients and microalgae, and to ensure the adequate dissolution of the injected gases as well as their rapid diffusion among microalgae. The recirculation speed can be changed during the conduction of the cultivation based on the needs of growth and conditioning.

### Gas Flow:

Depending on the conditions of growth or conditioning required, it is possible to proceed with mechanical (through run-off) or forced aeration, by introducing air or gas containing a certain portion of carbon dioxide (>0.01% at 100% v/v). The introduction of gas is necessary to supply the microalgae with the gaseous nutrients necessary for photosynthesis (carbon dioxide). The intake flow of gaseous nutrient must be effectively regulated to allow the effective dissolution of carbon dioxide and its bio-fixation in carbonaceous compounds by the photosynthetic activity of microalgae. The accurate calibration of the carbon dioxide flow has allowed reaching bio-fixation ratios >80% with the emission of gas composed at 99.999% by carbon dioxide.

### On-line Monitoring Parameters:

In order to optimize the cell growth rate (µ=g/L/d) and therefore the bio-fixation of carbon dioxide, the proposed example provides for the use of digital and analogue sensors to monitor the environmental parameters of the growth solution and the cell growth.

By way of example, but not exhaustively, the growth of Chlorella in a batch/ autotrophic condition involves the monitoring of: pH, dissolved carbon dioxide to evaluate the speed of introduction, dissolution in the aqueous growth solution and the consumption rate (bio-fixation), dissolved molecular oxygen to monitor the photosynthetic activity of microalgae, temperature of the nutrient solution, intensity and spectrum of the light incident on the panel, optical density (abs at 680 nm). The optical density data, shown on a graph in relation to the time of conduction of the cultivation, allows following the characteristic sigmoid pattern of cell growth in a closed batch system of microalgae. The parameters recorded by the sensors are used as early diagnostic elements for the evaluation of the state of growth and vitality of the microalgal biomass.

### Off-line Monitoring Parameters:

### Dry Weight - Biomass Concentration

In order to monitor the change in biomass concentration during the conduction of batch cultivation, it is possible to evaluate the dry weight of the microalgal biomass. A known and homogeneous culture volume can be sampled at regular intervals. The cells isolated and washed from the nutrient solution residues are dried (105 °C for 3 hrs). The concentration data (g/L), shown on a graph in relation to the time of conduction of the cultivation, allows following the characteristic sigmoid pattern of cell growth in a closed batch system of microalgae. Due to the time taken to carry out the evaluation of the dry weight, this parameter is considered, in the example shown, a less rapid diagnostic element compared to the previous parameters for the state of the system. The evaluation of the biomass concentration in the exemplary experiment recorded initial biomass of 0.2 g/L and minimum final biomass (after eleven days of cultivation) of 3 g/L.

### Released Gas Analysis - Bio-fixation Mass Balance

To realize the mass balance for the bio-fixation of the carbon dioxide injected into the photobioreactor, the proposed growth example of Chlorella includes gas analysis through a gas analyzer or gas-phase chromatography of the gases released by the system, net of the gas mass absorbed by the microalga to support its metabolic processes.

### Variations in the Conduction of the Cultivation System

The example shown illustrates the cultivation system of the microalga Chlorella in batch conditions. However, the cultivation, not only for the purposes of bio-fixation of carbon dioxide, can be carried out in semi-continuous (fed-batch) or continuous mode following the cell growth by means of a spectrophotometer, appropriately calculating the cell growth rate (µ=g/L/d) and preparing the necessary biomass discharges and the necessary additions of nutrient solution.

### Step 4. Collection of the biomass produced

The biomass produced in the closed growth system of the photobioreactor object of the patent can be collected at the end of the cultivation in batch condition or at periodic intervals in fed-batch and continuous mode. The collection techniques, by way of example and not exhaustively, include:
- filtration through sieving systems (for example, vibrating screens), traditional tangential (of ceramic, steel, plastic), or dynamic membrane filters;
- centrifugation
- flotation/ flocculation.

The biomass collected with the above-described exemplary methods does not undergo significant alterations in the chemical composition and may be destined to subsequent use.

### Example 2

For the present example, a batch culture of a photosynthetic micro-organism, in particular a photosynthetic cyanobacterium, for example, *Spirulina* sp., has been considered. Growth was carried out in totally autotrophic conditions aimed at the production of biomass and photostimulation for the accumulation of phycocyanin, a high added-value photosynthetic pigment.

The batch/ autotrophic cultivation of the cyanobacterium requires an aqueous growth solution that includes all inorganic minerals and the injection of a gas flow containing a smaller or larger fraction of carbon dioxide needed to meet the nutrition needs for the replication and growth. Moreover, the initial start of the cultivation of the photobioreactor object of the patent involves the use of a minimum quantity of starting biomass (inoculum), so that the initial concentration of the biomass has an optical density > 0.08 OD at 680 nm.

Based on the premises given above, the conduct of such cultivation involves the following steps:
1. Preparation of the biomass for the photobioreactor inoculum
2. Preparation of the photobioreactor for the conduction in batch/ autotrophic mode of cultivation
3. Conduction of cyanobacterium growth and photostimulation of phycocyanin accumulation
4. Collection of the biomass produced

### Step 1 - Preparation of the Biomass for the Photobioreactor Inoculum

The present step follows the operations of Example 1, with minor adaptations for the use of the cyanobacterium under examination. In particular, the Spirulina-Medium nutrient solution (modified by Aiba, S. & Ogawa, T. 1977) was used for the cultivation of Spirulina.

### Step 2. Preparation of the photobioreactor for the conduction in batch/autotrophic mode of cultivation

The preparation step of the photobioreactor for the production of Spirulina biomass follows the procedures described in Example 1, except the medium used for the growth of cyanobacterium, which in this case is "Spirulina-Medium".

### Step 3. Conduction of cyanobacterium growth and photostimulation of phycocyanin accumulation

The cyanobacterium biomass obtained with the previous operations of preparation of the inoculum (Step 1) is introduced into the photobioreactor object of the patent and prepared at the start of cultivation, as described in Step 2. During the growth period, it is possible to vary the composition of the nutrient solution to meet specific nutritional needs of the cyanobacterium or to modify the growth conditions of the accumulated biomass through the introduction of new nutrients into the photobioreactor.

For the conduction of cultivation in batch/ autotrophic conditions, it is necessary to control and monitor the chemical/physical parameters within the system which, in the example shown, reflect the growth needs of the selected cyanobacterium, Spirulina.

### Lighting:

Growth is carried out under controlled and modulated lighting according to the terms described in the patent. It is possible to adjust the intensity between 10-500 µmol m⁻² s-¹ Photosynthetic Photon Flow Density (PPFD), with or without day-night cycle. Furthermore, it is possible to qualitatively modify the lighting spectrum according to the absorption requirements of the cultivated alga and the desired growth and conditioning needs. In the case described, the growth is carried out using the lighting spectrum characteristic of the WHITE LED with a continuous lighting cycle (24h/24h) in order to maximize the biomass accumulation speed in the photobioreactor and reach the final step of exponential growth in less time. At the end of the exponential growth step, the lighting spectrum of the photobioreactor is modified qualitatively by exposing the cyanobacterium to the light lengths of the area corresponding to the red and deep red (640-800 nm) of the visible light spectrum. The conditioning applied to the cyanobacterium induces photostimulation of the phycocyanin accumulation as a photosynthetic pigment until the stationary phase of growth characteristic of the sigmoid curve is reached.

### Flow of the Nutrient Solution:

The recirculation and handling system of the nutrient solution (hydraulic recirculation pump) is equipped with a speed regulation system. The speed of the nutrient solution recirculation system must be adjusted appropriately in a range that can vary from 5 to 20 L/min in order to guarantee the adequate frequency of exposure of the cyanobacteria, in a way similar to Example 1. The recirculation speed can be changed during the conduction of the cultivation based on the needs of growth and conditioning.

### Gas Flow:

Depending on the conditions of growth or conditioning required, it is possible to proceed with mechanical (through run-off) or forced aeration, by introducing air or gas containing a certain portion of carbon dioxide (> 0.01% at 100% v/v). The introduction is necessary to supply the cyanobacterium with the gaseous nutrients necessary for photosynthesis (carbon dioxide). The inflow of gaseous nutrients must be regulated and positioned effectively to allow the effective dissolution of carbon dioxide, minimizing gas consumption, but ensuring the minimum requirement for optimal growth of the microorganism.

### On-line Monitoring Parameters:

In order to optimize the cell growth rate (µ=g/L/d), the proposed example provides for the use of digital and analogue sensors to monitor the environmental parameters of the growth solution and the cell growth similarly to Example 1.

### Off-line Monitoring Parameters:

### Dry Weight - Biomass Concentration

In order to monitor the change in biomass concentration during the conduction of batch cultivation, it is possible to evaluate the dry weight of the microalgal biomass similarly to Example 1. The evaluation of the biomass concentration in the exemplary experiment recorded initial biomass of 0.1 g/L and minimum final biomass (after eleven days of cultivation) of 5.3 g/L.

### Step 4. Collection of the biomass produced

The biomass produced in the closed growth system of the photobioreactor object of the patent can be isolated and collected at the end of the cultivation in batch condition, at periodic intervals in fed-batch and continuous mode for the continuous mode. The collected biomass, with a high phycocyanin content, can be used for subsequent processing. The collection techniques, by way of example and not exhaustively, are shown in Example 1.

To demonstrate the effectiveness of the present invention, experimental tests have been carried out and the data specified below have been collected; these data allow to compare the effectiveness of the present invention with respect to the known solutions, in particular with respect to the invention of the European Patent no. EP 2 830 413 B1. In particular, given the same algal species, comparative data are shown below between fluorescent light with neon and LED lighting, energy consumption savings, higher productivity/lower growth time depending on the variation in the quality/quantity of the light.

The growth of Tetradesmus obliquus (Turpin) (for example, Scenedesmus obliquus (Turpin) Kützing) in a planar photobioreactor, which refers to the invention of the European Patent no. EP 2 830 413 B1, was compared with the growth of the same green microalga in a photobioreactor with LED lighting object of the present invention, maintaining the same conditions: composition of the culture medium, pH, temperature, day-night lighting cycle, CO₂ supply, flow. In order to obtain an objective comparison between the two cultivation systems, the light intensity (expressed as µmol/m⁻²/ s⁻¹ Photosynthetic Photon Flow Density) in the two growth conditions was kept equal for the entire conduction of experiments.

The results obtained, expressed as "Growth Rate" (g/L/day) showed an average value of 0.02 g/L/day in the case of neon lighting in the photobioreactor according to the invention of European Patent no. EP 2 830 413 B1 and an average value equal to 0.05 g/L/day in the photobioreactor with LED lighting, object of the present invention. From this, it can be seen that, under the same growing conditions, the reproduction speed is more than twice as high.

As mentioned above, the present invention has been compared with the invention of the European Patent no. EP 2 830 413 B1, with the same algal species, in terms of power consumption savings and greater productivity/less growth time depending on the variation of the quality/quantity of the light, which were determined as described below.

### Measurement of power consumption savings

The power consumed by the illuminators was measured by using an amperometric clamp on two systems operating under the following conditions:
- lighting with three 58 W neon tubes and use of a configuration on the LED illuminator such as to emit the same amount of light expressed in active photosynthetic radiation by the neon-illuminated system identified in 40 PAR;
- lighting with five 58 W neon tubes and use of a configuration on the LED illuminator such as to emit the same amount of light expressed in active photosynthetic radiation by the neon-illuminated system identified in 76 PAR.

The comparison between the two systems resulted in a power absorption which for the three-neon tubes system is equal to 174 W, while for the LED system is equal to 64 W, for the five-neon tubes system is equal to 290 W, while for the LED system is equal to 106 W. In both cases, savings amount to a reduction in power consumption of over 60%.

### Measurement of Productivity according to the Variation of the Quantity of Light

From the tests carried out for the evaluation of power savings reported above, the parameters of growth and productivity have also been inferred. The comparison is reported under equal conditions of the two lighting systems in the 40 PAR configuration. In this case, with neon lighting, an increase of 43% in biomass in 5 days is obtained while using the LED lighting system, the increase in biomass over the same period is equal to 66%. The comparison is carried out only on white light, as traditional neon systems do not allow to influence the composition of the spectrum.

The results of the comparison are summarized in the Table below.

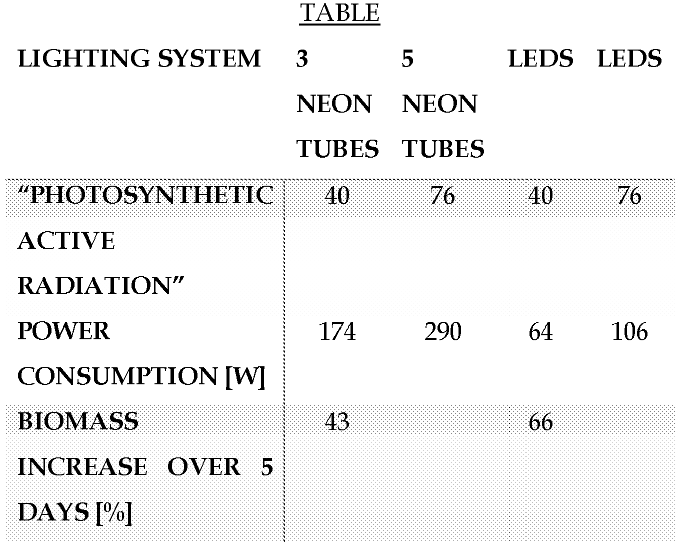

The aforementioned Table shows that, under the same conditions in the most economical energy-saving scenario, the LED lighting system consumes about 60% less than the traditional lighting system. Reduction that does not result into a decrease in biomass production since, under the same growth conditions, the LED lighting system over 5 days of observation has a productivity of about 20% more than the traditional system.

It is, therefore, apparent that the technical solution according to the present invention is capable of achieving the stated and expected results.

As it can be deduced from the foregoing, the innovative technical solution described herein has the following advantageous features:
- optimizing the growth processes of photosynthetic organisms;
- reducing the growth processes of photosynthetic organisms;
- decreasing the consumption of both nutrients and power;
- realizing the contiguity with natural lighting to allow a bright exposure throughout the day;
- realizing light stress scenarios to make photosynthetic organisms produce secondary products with a high-yield.

From the description above it is, therefore, apparent how the apparatus and method according to the present invention allow to achieve the intended objects.

Therefore, it is apparent to a person skilled in the art that it is possible to make modifications and further variants to the solution described with reference to the accompanying figures, without departing from the teaching of the present invention and the scope of protection, as defined by the appended claims.

## Claims

1. An apparatus (100, 100') for the culture of photosynthetic microorganisms (50, 50') comprising
- at least one first panel (20, 20') containing said photosynthetic microorganisms (50, 50') inside it within a circuit having an input and an output; and
- at least one device for artificial lighting (10, 10') of said photosynthetic microorganisms (50, 50'), wherein
- said at least one device for artificial lighting (10, 10') is an optical device and comprises
▪ a panel having an optical guide (1, 1');
▪ a plurality of LEDs (2, 2'); and
▪ a regulation and control panel (4, 4') of said at least one device for artificial lighting (10, 10'),
- said at least one regulation and control panel (4,4') is suitable to vary both the spectrum, between 200 and 900 nm in order to optimize both the quality of the light emitted, and the light intensity, between 1% and 100% in order to obtain emission uniformity, through the optical guide (1, 1'); and
- the first panel and the panel with the optical guide are facing one another.

2. The apparatus (100, 100') for the culture of photosynthetic microorganisms (50, 50') according to claim 1, wherein said at least one device for artificial lighting (10, 10') further comprises
▪ a pair of profiles (3, 3') to dissipate the thermal energy generated by the LEDs (2, 2');
▪ a plurality of shielded cables (5, 5') for the connection between the LEDs (2, 2') and the regulation and control panel (4, 4');
▪a pair of supports (6, 6') for centering the optical guide (1, 1');
▪ a pair of uprights (7, 7') to guarantee the rigidity of said at least one device for artificial lighting (10, 10');
▪ closing plugs (8, 8') to close the uprights (7, 7'); and
▪ at least one power supply system (9, 9').

3. The apparatus (100) for the culture of photosynthetic microorganisms (50, 50') according to claim 1 or 2, wherein said at least one device for artificial lighting (10) is a mono-emissive optical device.

4. The apparatus (100') for the culture of photosynthetic microorganisms (50, 50') according to claim 1 or 2, further comprising at least a second panel (30') containing said photosynthetic microorganisms (50, 50') inside it and wherein said at least one device for artificial lighting (10') is a bi-emissive optical device.

5. The apparatus (100, 100') according to claim 3 or 4, wherein said at least one mono-emissive (10) or bi-emissive (10') optical device for artificial lighting is placed in direct contact with said at least first panel (20, 20').

6. The apparatus (100, 100') according to any of the preceding claims, wherein said at least one regulation and control panel (4, 4') comprises
▪ a driver for the current driving of the LEDs (2, 2');
▪ a power supply stage;
▪ a programmable microprocessor; and
▪ a digital control interface.

7. The apparatus (100, 100') according to claim 6, wherein the spectrum ranges between 200 and 900 nm and wherein the light intensity ranges between 1% and 100%, the spectrum and the light intensity dynamically varying according to the operating specifications of the device for artificial lighting (10, 10').

8. A method for the culture of photosynthetic microorganisms (50, 50') comprising the following steps:
- preparing an apparatus (100, 100') for the culture of photosynthetic microorganisms (50, 50') according to any claim 1 to 7 (step 200);
- activating said at least one device for artificial lighting (10, 10') of said photosynthetic microorganisms (50, 50') and actuating said at least one regulation and control panel (4, 4') so as to vary both the spectrum, between 200 and 900 nm thus optimizing the quality of the light emitted, and the light intensity, between 1% and 100% thus obtaining emission uniformity (step 201); and
- artificially illuminating said at least one first panel (20, 20') containing said photosynthetic microorganisms (50, 50') inside it for a predetermined time, thus reaching the desired accretion level of said photosynthetic microorganisms (50, 50') (step 202).

9. The method according to claim 8, wherein:
- said at least one regulation and control panel (4, 4') is operated so as to vary the spectrum between 200 and 900 nm and the light intensity between 1% and 100%, the spectrum and the light intensity dynamically varying according to the operating specifications of the device for artificial lighting (10, 10').

10. The method according to claim 8 or 9, wherein said photosynthetic microorganisms are selected from green algae (Chlorophyta), diatoms (Chrysophyta), dinoflagellates (Dinophyta), red microalgae (Rhodophyta), protists and photosynthetic bacteria such as cyanobacteria.

11. The method according to claim 10, wherein said photosynthetic microorganisms are selected from Chlorella sp., Cyanidium sp., Euglena sp. and Spirulina sp..

12. The method according to any claim 8 to 11, wherein the predetermined period of time is equal to or greater than two days, and wherein the desired level of growth of said photosynthetic organisms (50, 50') is equal to or greater than a corresponding growth rate (µ) equal to 0.01 g/L/day.

13. The method according to any claim 8 to 12, wherein said apparatus (100, 100') is at ambient pressure or under pressure.

## Patentansprüche

1. Vorrichtung (100, 100') zur Wachstum von photosynthetischen Mikroorganismen (50, 50'), umfassend:
- mindestens ein erstes Panel (20, 20'), das die photosynthetischen Mikroorganismen (50, 50') in ihrem Inneren innerhalb eines Kreislaufs enthält, der mit einem Einlass und einem Auslass versehen ist; und
- mindestens eine Vorrichtung zur künstlichen Beleuchtung (10, 10') von photosynthetischen Mikroorganismen (50, 50'), wobei
- mindestens die Vorrichtung zur künstlichen Beleuchtung (10, 10') ist eine optische Vorrichtung und umfasst:
* ein mit einem optischen Leiter (1, 1') ausgestattetes Panel
* eine Mehrzahl von LEDs (2, 2'); und
* ein Regel- und Bedienpanel (4, 4') mindestens einer Vorrichtung zur künstlichen Beleuchtung (10, 10')
- wobei das mindestens eine Regel- und Bedienpanel (4, 4') in der Lage ist, das Spektrum zwischen 200 und 900 nm zu variieren, um sowohl die Qualität des emittierten Lichts als auch die Lichtintensität zwischen 1 % und 100 % zu optimieren, um eine gleichmäßige Emission durch den optischen Leiter (1, 1') zu erreichen; und
- wobei das erste Panel und das Panel mit dem optischen Leiter einander zugewandt angeordnet sind.

2. Vorrichtung (100, 100') zur Kultivierung photosynthetischer Mikroorganismen (50, 50') nach Anspruch 1, wobei die mindestens eine Vorrichtung zur künstlichen Beleuchtung (10, 10') weiterhin umfasst
- ein Paar von Profilen (3, 3') zur Ableitung der von den LEDs (2, 2') erzeugten Wärmeenergie;
- eine Mehrzahl von abgeschirmten Kabeln (5, 5') für die Verbindung zwischen den LEDs (2, 2') und dem Bedien- und Regelpanel (4, 4');
- ein Paar Stützen (6, 6') zur Zentrierung des optischen Leiters (1, 1');
- ein Paar Pfosten (7, 7'), um die Steifigkeit mindestens einer Vorrichtung zur künstlichen Beleuchtung (10, 10') zu gewährleisten;
- Verschlusskappen (8, 8') zum Verschließen der Pfosten (7, 7'); und
- mindestens ein Energieversorgungssystem (9, 9').

3. Vorrichtung (100) zur Kultivierung photosynthetischer Mikroorganismen (50, 50') nach Anspruch 1 oder 2, wobei die mindestens eine Vorrichtung zur künstlichen Beleuchtung (10) eine optische Einzelemissionsvorrichtung ist.

4. Vorrichtung (100) zur Kultivierung photosynthetischer Mikroorganismen (50, 50') nach Anspruch 1 oder 2, mindestens ein zweites Panel (30') aufweisend, das im Inneren photosynthetische Mikroorganismen (50, 50') enthält und worin die mindestens eine Vorrichtung für künstliche Beleuchtung (10') eine optische Doppelemissionsvorrichtung ist.

5. Vorrichtung (100, 100') nach Anspruch 3 oder 4, wobei die mindestens eine optische Einzelemissionsvorrichtung (10) oder Doppelemissionsvorrichtung (10') für künstliche Beleuchtung in direktem Kontakt mit mindestens einem ersten Panel (20, 20') angeordnet sind.

6. Vorrichtung (100, 100') nach einem der vorhergehenden Ansprüche, wobei mindestens ein Regel- und Bedienpanel (4, 4') mindestens
- eine Führung zur Stromleitung durch die LEDs (2, 2');
- einen Energieversorgungsschritt;
- ein programmierbarer Mikroprozessor; und
- eine digitale Bedienschnittstelle,
umfasst.

7. Vorrichtung (100, 100') nach Anspruch 6, wobei das Spektrum zwischen 200 und 900 nm und die Lichtintensität zwischen 1 % und 100 % variiert, wobei das Spektrum und die Lichtintensität dynamisch entsprechend der Betriebsdaten der Vorrichtung zur künstlichen Beleuchtung (10, 10') variieren.

8. Verfahren zur Kultivierung photosynthetischer Mikroorganismen (50, 50') umfassend die folgenden Schritte:
- Bereitstellen einer Vorrichtung (100, 100') zur Kultivierung photosynthetischer Mikroorganismen (50, 50') nach einem der Ansprüche 1 bis 7 (Schritt 200);
- Aktivieren der mindestens einen Vorrichtung zur künstlichen Beleuchtung (10, 10') der photosynthetischen Mikroorganismen (50, 50') und aktivieren der mindestens eines Regel- und Steuerpanels (4, 4'), um sowohl das Spektrum zwischen 200 nm und 900 nm, wodurch die Qualität des emittierten Lichts optimiert wird, und die Lichtintensität zwischen 1 % und 100 %, zu variieren, wodurch eine Emissionsgleichmäßigkeit optimiert wird (Schritt 201); und
- Künstliches Beleuchten mindestens eines ersten Panels (20, 20'), das darin enthaltene photosynthetische Mikroorganismen (50, 50') für eine vorgegebene Zeit enthält, um so das gewünschte Wachstumsniveau der photosynthetischen Mikroorganismen (50, 50') (Schritt 202) zu erreichen.

9. Verfahren nach Anspruch 8, wobei:
- mindestens ein Regel- und Bedienpanel (4, 4') zur Variation des Spektrums zwischen 200 und 900 nm und der Lichtintensität zwischen 1 % und 100 % betätigt wird, wobei sich das Spektrum und die Lichtintensität entsprechend dynamisch die Betriebsdaten der Vorrichtung zur künstlichen Beleuchtung (10, 10'), variieren.

10. Verfahren nach Anspruch 8 oder 9, wobei die photosynthetischen Mikroorganismen aus Grünalgen (Chlorophyten), Diatomeen (Chrysophyten), Dinoflagellaten (Dynophyten), roten Mikroalgen (Rhodophyten), Protisten und photosynthetischen Bakterien wie Cyanobakterien, ausgewählt sind.

11. Verfahren nach Anspruch 10, wobei die photosynthetischen Mikroorganismen aus Chlorrela sp., Cyanidium sp., Euglena sp. end Spirulina sp., ausgewählt sind.

12. Verfahren nach einem der Ansprüche 8 bis 11, wobei der vorgegebene Zeitraum gleich oder größer als zwei Tage ist und wobei der gewünschte Wachstumsstand der photosynthetischen Organismen (50, 50') gleich oder größer als eine entsprechende Wachstumsrate (u) von 0,01 g/L/Tag ist.

13. Verfahren nach einem der Ansprüche 8 bis 12, wobei sich die Vorrichtung (100, 100') auf Umgebungsdruck oder unter Druck befindet.

## Revendications

1. Dispositif (100, 100') de culture de microorganismes photosynthétiques (50, 50') comprenant :
- au moins un premier panneau (20, 20') contenant les microorganismes photosynthétiques (50, 50') à l'intérieur, au sein d'un circuit muni d'une entrée et d'une sortie; et
- au moins un dispositif d'éclairage artificiel (10, 10') des microorganismes photosynthétiques (50, 50'), où
- au moins un dispositif d'éclairage artificiel (10, 10') est un dispositif optique et comprend:
* un panneau équipé d'un guide optique (1, 1')
* une pluralité de LED (2, 2'); et
* un panneau de réglage et commande (4, 4') d'au moins un dispositif d'éclairage artificiel (10, 10')
- au moins un panneau de réglage et commande (4, 4') pouvant modifier le spectre entre 200 et 900 nm, afin d'optimiser à la fois la qualité de la lumière émise et l'intensité lumineuse entre 1% et 100%, afin d'obtenir une uniformité d'émission à travers le guide optique (1, 1'); et
- le premier panneau et le panneau avec le guide optique étant disposés face à face.

2. Dispositif (100, 100') de culture de microorganismes photosynthétiques (50, 50') selon la revendication 1, **caractérisé en ce qu'**au moins un dispositif d'éclairage artificiel (10, 10') comprend en outre
- une paire de profilés (3, 3') pour dissiper l'énergie thermique générée par les LED (2, 2');
- une pluralité de câbles blindés (5, 5') pour la connexion entre les LED (2, 2') et le panneau de réglage et commande (4, 4');
- une paire de supports (6, 6') pour centrer le guide optique (1, 1');
- une paire de montants (7, 7') pour garantir la rigidité d'au moins un dispositif d'éclairage artificiel (10, 10');
- des bouchons de fermeture (8, 8') pour fermer les montants (7, 7'); et
- au moins un système d'alimentation en énergie (9, 9').

3. Dispositif (100) de culture de microorganismes photosynthétiques (50, 50') selon la revendication 1 ou 2, **caractérisé en ce qu'**au moins un dispositif d'éclairage artificiel (10) est un dispositif optique à simple émission.

4. Dispositif (100) de culture de microorganismes photosynthétiques (50, 50') selon la revendication 1 ou 2, comprenant en outre au moins un deuxième panneau (30') contenant les microorganismes photosynthétiques (50, 50') à l'intérieur et dans lequel au moins un dispositif d'éclairage artificiel (10') est un dispositif optique à double émission.

5. Dispositif (100, 100') selon la revendication 3 ou 4, dans lequel au moins un dispositif optique d'éclairage artificiel à simple émission (10) ou à double émission (10') est placé en contact direct avec au moins le premier panneau (20, 20').

6. Dispositif (100, 100') selon l'une des revendications précédentes, dans lequel au moins un panneau de réglage et commande (4, 4') comprend
- un guide pour la conduction d'un courant à travers les LED (2, 2');
- un étage d'alimentation en énergie;
- un microprocesseur programmable; et
- une interface de commande numérique.

7. Dispositif (100, 100') selon la revendication 6, dans lequel le spectre varie entre 200 et 900 nm et dans lequel l'intensité lumineuse varie entre 1 % et 100 %, dans lequel le spectre et l'intensité lumineuse varient dynamiquement en fonction des spécifications de fonctionnement du dispositif d'éclairage artificiel (10, 10').

8. Procédé de culture de microorganismes photosynthétiques (50, 50') comprenant les étapes suivantes :
- préparer un dispositif (100, 100') de culture de microorganismes photosynthétiques (50, 50') selon l'une des revendications 1 à 7 (étape 200) ;
- activer au moins un dispositif d'éclairage artificiel (10, 10') des microorganismes photosynthétiques (50, 50') et activer au moins un panneau de réglage et commande (4, 4') de manière à faire varier à la fois le spectre, entre 200 et 900 nm, optimisant ainsi la qualité de la lumière émise, soit l'intensité lumineuse entre 1% et 100%, optimisant ainsi une uniformité d'émission (étape 201); et
- éclairer artificiellement au moins un premier panneau (20, 20') contenant les microorganismes photosynthétiques (50, 50') à l'intérieur pendant une durée prédéterminée, atteignant ainsi le niveau de croissance souhaité des microorganismes photosynthétiques (50, 50') (étape 202).

9. Procédé selon la revendication 8, dans lequel:
- au moins le panneau de réglage et commande (4, 4') est actionné pour faire varier le spectre entre 200 et 900 nm et l'intensité lumineuse entre 1% et 100%, le spectre et l'intensité lumineuse variant dynamiquement en fonction des spécifications de fonctionnement du dispositif d'éclairage artificiel (10, 10').

10. Procédé selon la revendication 8 ou 9, dans lequel les microorganismes photosynthétiques sont choisis parmi les algues vertes (chlorophytes), les diatomées (chrysophytes), les dinoflagellés (dynophytes), les microalgues rouges (rhodophytes), les protistes et les bactéries photosynthétiques telles que les cyanobactéries.

11. Procédé selon la revendication 10, dans lequel les microorganismes photosynthétiques sont choisis entre Chlorrela sp., Cyanidium sp., Euglena sp. et Spiruline sp.

12. Procédé selon l'une des revendications 8 à 11, dans lequel la période de temps prédéterminée est égale ou supérieure à deux jours, et dans lequel le niveau de croissance souhaité des organismes photosynthétiques (50, 50') est égal ou supérieur à un taux de croissance correspondant (µ) égal à 0,01 g/L/jour.

13. Procédé selon l'une des revendications 8 à 12, dans lequel le dispositif (100, 100') est à pression ambiante ou sous pression.
